# EUROPEAN PATENT SPECIFICATION

(11) **EP 2 402 757 B1**
(45) Date of publication and mention of the grant of the patent: **16.11.2016**
(21) Application number: 11166863.8
(22) Date of filing: 20.05.2011
(51) Int. Cl.: G01N 33/567

(54) **A method of enriching spermatozoa of mammals bearing X-chromosome or Y-chromosome**
Verfahren zur Anreicherung von Spermatozoiden in Säugern mit dem X-Chromosom oder Y-Chromosom
Procédé d'enrichissement des spermatozoïdes de mammifères contenant le chromosome X ou le chromosome Y

(30) Priority: 29.06.2010 BR PI0401765
(43) Date of publication of application: 04.01.2012
(73) Proprietor: HY Biotecnológica Ltda., 28030-035 Campos dos Goytacazes (BR)
(72) Inventor: Matta, Marcos Fernando Resende, 28030-035 Campos dos Goytacazes - RJ (BR)
(74) Representative: Winter, Brandl, Fürniss, Hübner, Röss, Kaiser, Polte - Partnerschaft mbB

(56) References cited:
- US-A- 4 680 258
- US-A- 5 840 504
- US-A1- 2002 115 055
- PIEDRAHITA J A ET AL: "INVESTIGATION OF SPERM CYTOTOXICITY AS AN INDICATOR OF ABILITY OF ANTISERA TO DETECT MALE-SPECIFIC ANTIGEN ON PREIMPLANTATION MOUSE EMBRYOS", JOURNAL OF REPRODUCTION AND FERTILITY, JOURNALS OF REPRODUCTION AND FERTILITY LTD, GB, vol. 74, no. 2, 1 January 1985 (1985-01-01), pages 637-644, XP009151830, ISSN: 0022-4251, DOI: 10.1530/JRF.0.0740637
- ELLEN H. GOLDBERG ET AL: "Serological Demonstration of H-Y (Male) Antigen on Mouse Sperm", NATURE, vol. 232, no. 5311, 13 August 1971 (1971-08-13), pages 478-480, XP055006319, ISSN: 0028-0836, DOI: 10.1038/232478a0
- BENNETT D ET AL: "Sex ratio in progeny of mice inseminated with sperm treated with H-Y antiserum", NATURE, NATURE PUBLISHING GROUP, LONDON, GB, vol. 246, no. 5431, 30 November 1973 (1973-11-30), pages 308-309, XP009151829, ISSN: 0028-0836, DOI: 10.1038/246308A0

## Description

### Field of the Invention

The present invention relates to an improvement in the invention described and claimed in Brazilian patent application BRP10401765-0 and is based on the use of monoclonal antibodies directed against the genus-specific proteins located in the cytoplasm membrane of spermatozoa associated to the action of the classical complement pathway, in order to increase the percentage of one of the genders in the offspring of mammals.

Said Brazilian patent application BRP10401765-0 discloses immunological processes for selecting spermatozoa having X-chromosome or Y-chromosome, including the action of genus-specific monoclonal antibodies, associated to the complement action through the classic route. The present invention relates to an improvement in the form of eliminating the alternative complement pathway.

### Foundation of the Invention

Producers of dairy cattle and cattle for slaughter prefer animals of a given sex. Producers of dairy cattle have greater interest in females. Producers of cattle for slaughter prefer males due to the greater gain in weight while they grow up and by virtue of the quality of the flesh. By selecting the sex, a breeder has the option of choosing the desired sex with greater 50-50 chance of obtaining the desired sex.

In general, a producer has about 40-50% of animals to be selected genetically for replacement of the breeding cows in the herd. With sexing this percentage rises to 80-90% of females, causing the selection to be carried out more intensively and in a more efficient manner.

Another important factor in sexing spermatozoa when producing embryos is the better utilization of ovocytes, since they are fertilized with the spermatozoon having the desired chromosome. In this way, there will be better utilization of donors and recipients, factor of great financial repercussion.

The method of sexing spermatozoa can be used for all the species and mainly those of zootechnical interest, such as cattle, sheep, equines and swine. For this purpose, it is enough for the monoclonal antibody to recognize the genus-specific proteins.

It was found that the way of eliminating the alternative pathway by heating the serum of the guinea-pig up to 52°C for thirty minutes did not have a constant response, and the variation observed can be attributed to the elimination of some proteins that are necessary to the classical complement pathway or to the complete elimination of the B protein, which accounts for the evaluation of the alternative complement pathway. Since the complement system has enzymes that are extremely sensitive to temperature, the heating of the serum up to 52°C for thirty minutes, with subsequent cooling to 37° C, has exhibited variable results and with undesirable reproducibility.

The present improvement discloses a more advantageous and effective manner of eliminating the alternative complement pathway by dilution that limits the source of complement (guinea-pig's serum) without any other treatment for eliminating the alternative pathway.

### Background of the Invention

In an ejaculate, semen has approximately an equal number of spermatozoa possessing X-chromosomes and Y-chromosomes. Fertilization of ovocytes with spermatozoa having Y-chromosome will produce male embryos, while spermatozoa having X-chromosomes will give rise to female embryos.

A number of techniques have been tested for sexing spermatozoa. However, none of them proved to be effective for producing this separation of spermatozoa in number and quality suitable for use by artificial insemination.

The first methodologies used for obtaining fractions rich in spermatozoa having X- or Y-chromosomes were separation by motility (Kaiser, G.P.R. et al, 1974 - Relative increase in Y-chromatin-Bearing spermatozoa after in vitro penetration into human cervical mucus. IRCS, 2, p. 1100) and sedimentation by density (Soupart, P., 1975, MGA-M-appearance in ejaculated human sperm. Eight Ann. Meeting Society Study Reprod., Fort Collins, Co. Abstr. 133). These methods assume that a spermatozoon having the Y-chromosome is less dense and has greater motility. However, due to the morphological variety of the spermatozoa, these techniques are not successful in enriching protozoa populations.

Another method of separating spermatozoa is based on the quantitative difference of DNA present in spermatozoa having X- or Y-chromosomes. Spermatozoa having X-chromosomes have about 4% more DNA than spermatozoa having Y-chromosomes. By using these data, researchers have been working with a view to separate spermatozoa by flow cytometry. The main problem of this technique is the impossibility of obtaining large amounts of sexed semen for artificial insemination programs. In addition to this factor, when the spermatozoa are separated by flow cytometry, they go through the "cell sorter" at a speed of approximately 100 km/h, which causes numberless lesions to the spermatozoa. These problems make sexing difficult for large-scale use, resulting in a technique that can be used in a much reduced number of bulls and with great restrictions regarding the quality of the spermatozoa.

Immunological methods have also been used for separating X-and Y-spermatozoa. These methods are based on the fact that spermatozoon RNA polymerase is able to transcribe different membrane proteins in haploid cells (Moore, G.P.M., 1971, DNA-dependent RNA syinthesis in fixed cells during spermatogogenesis in mouse. Exptl. Cell Research, 68: 462-465). Thus, the different antigens present in on the surface of the different populations of spermatozoa could be used for separation. However, it is known that the H-Y antigen is little immunogenic, and to some methodologies may not be sufficiently sensitive for spermatozoa sexing.

Some researchers further suggest that the Y-spermatozoon does not synthesize the H-Y protein, but absorbs it into its surface (Garner, D.L. et al, 1984. An overview of separation of X- and Y-spermatozoa. Proceedings of the Tenth Technical Conference on Artificial Insemination and Reproduction (National Association of Animal Breeders), p. 87-92). According to this statement, Hoope and Koo (Hoope, P. and Koo, G.C., 1984, Reacting mouse sperm cells with monoclonal H-Y antibodies does not influence Sex ratio of eggs fertilized in vitro, J. Rep. Immunol, 6:1-9) show that the X- and Y-spermatozoa react with anti-H-Y antibody. However, these researchers demonstrate that the ability of these antibodies decline with maturity of the spermatozoon cells, implying an effect masking the regulation of the gene expression. Another possibility is that the technique used and its experimental outlining fail to demonstrate the presence of the antigen due to the too low immunogenicity thereof (Moore, D.H. and Gledhill, B.L., 1988. How large should my study be so that I can detect an altered Sex ratio? Fertility and Sterility, 50:21-25).

Silvers et al (Silvers, W.K. ; Gasser, D.L. ; Eicher, E.M., 1982. H-Y antigen, serologically detectable male antigen and Sex determination. Ce//, 28,439) have suggested, on the basis of experiments with transplant rejection, that the H-Y antigen is serologically detectable. These researches have introduced the designation SDMA (serologically defined male antigen). These results have been confirmed in various laboratories, by using a variety of methods and different types of specific male antibodies (Reilly, B.D. and Goldberg, E. H., 1991. Evaluation of a solid-phase cellular enzyme immunoassay for detection of the serologically defined male antigen. J. Immunol. Methods, 142: 121-126).

Hendriksen P. J. M. (1999) have suggested that dead spermatozoa promote a protein reversion, exhibiting a superimmunogenic antigen, against which most monoclonal antibodies react strongly.

Immunological methodologies for increasing the percentage of subjects of a given sex in mammals were described in the patents of Bryan, US 4,919,749 and US 4,448,767. The method disclosed in these patents involve the use of the specific male antibody coupled to an immunoabsorbent material in solid phase to carry out separation of sex-determining spermatozoa contained in a sperm suspension. Although these two methodologies described by Bryan serve to increase the percentage of offspring of the desired sex, the methods described by these authors have drawbacks, since they are complex and low-sensitivity methodologies. In the animal immunization with specific male antigen, the antibody produced does not have highly specific binding to the H-Y antigen found in male spermatozoa, there being low proportion of spermatozoa of female sex with respect to the objective.

Pending Brazilian application BRP19704313-3, of the inventors of the present invention, used hen-egg immunoglobulin associated to mono-clonal antibodies against sex-specific antigens in immunosexing of bovine spermatozoa. The technique proposes that the sexing should be carried on the basis of the technique of agglutination of spermatozoa bearing the HY antigen by the action of the monoclonal antibody associated to the action of a hen-specific antibody against the isotype of the monoclonal antibody.

Methods of sexing spermatozoa have also been proposed in patent application US20090208977. This application proposes sexing based on the binding of the monoclonal antibody to the genus-specific antigen and separation of the spermatozoa by using solid supports such as magnetic microbeads.

Patent US6489092 also discloses methods for increasing the percentage of a given gender in the offspring of mammals through immunologic methods that use non-porous magnetic beads for the separation of spermatozoa.

All the techniques mentioned above had, as a negative factor for being successful, little immunogenicity of the HY antigen and the force with which the spermatozoa move, thus preventing the antigen-antibody bindings from being sufficiently strong for achieving the objective.

The binding of monoclonal antibodies associated to the classical complement pathway is the most sensitive and more specific method possible for carrying out the immunological sexing of spermatozoa. The sensitivity of the technology of fixing the complement is hundreds of times higher than the technology of agglutination, and the need to form the antigen-antibody complex in order to open the receptor for activation of the classical complement pathway makes this methodology more specific among all those described.

Antibodies of mammals are capable of activating the classical complement pathway present in the serum of various other species of mammals. The alternative complement pathway does not need the presence of antibodies for acting and, due to this fact, it acts indiscriminately on all the spermatozoa. The actuation of the alternative pathway is the probable cause of loss of efficacy of the previous methods which do not use a way of inhibiting the alternative complement pathway.

US 2002/115055 A1 describes a method of enriching spermatozoa of mammals bearing an Y-chromosome comprising incubating an antibody against a male-specific cell surface protein on spermatozoa with a mixture of bovine spermatozoa and heated guinea pig serum.

Therefore, there is the need in the technique for obtaining manners of inhibiting effectively the alternative complement pathway for use of the method of sexing spermatozoa on mammals.

### Brief Description of the Invention

The present invention relates to an improvement of the invention described in BRP10401765-0, which provides a sexing method which differentiates the gender of bovine spermatozoa by using specific monoclonal antibodies for the H-Y antigen or the H-X antigen and actuating the classical complement pathway.

In an embodiment of the invention, the method of enriching spermatozoa of mammals bearing X-chromosome or Y-chromosome involves the binding of monoclonal antibodies specific to the H-Y antigen or the H-X antigen located on the surface of the cytoplasm membrane of the spermatozoa bearing Y-chromosome or X-chromosome, wherein said binding includes the activation of the classical complement pathway and the eliminating of the action of alternative complement pathway by limiting dilution of the complement source, wherein the complement source is a guinea-pig's serum.

In a preferred embodiment, the limit dilution of the complement source takes place for dilution at a concentration of 0.8%.

Inhibitors of the alternative pathway, such as antibodies against properdin, against protein B or against any other protein or protein complex or amino acid complex capable of inhibiting the alternative complement pathway may be used.

Among the embodiments of the method of the invention, one can further use a second antibody that recognizes the genus-specific monoclonal antibody, thus increasing the binding sites of the classical alternative complement pathway.

Kits for use in the methods of the invention are also disclosed.

### Brief Description of the Figures

Figure 1 represents the electrophoresis in gel in SDS-polyacrylamide used for separating the proteins extracted from spermatozoon membrane. BSA was used as a standard. Two bends with molecular mass of about 19 kDa were purified from gel, quantified and used for immunizing mice Balb/c for the production of monoclonal antibodies by using the standard technique to obtain hybridomas.
Figure 2 shows histological sections of the spleen showing the reactivity of antibody C11 F clones to spleen proteins of male and of female.
Figure 3 shows histological sections of liver showing the reactivity of antibody C11 F clones to liver proteins of male and of female.
Figure 4 shows histological sections of kidney showing the reactivity of antibody C11 F clones to kidney protein of male and of female.
Figure 5 represents the standard in SDS-PAGE of spermatozoa sexed by the action of monoclonal antibodies and classical complement pathway.
Figure 6A shows the histogram of the analysis of flow cytometry of spermatozoa marked only with the anti-HY antibody (C11 F), without fluorescein, showing the position of the unmarked cells.
Figure 6C shows the histogram of the analysis of flow cytometry of the populations marked with monoclonal anti-HY antibody (C11 F) and secondary anti-mouse goat antibody marked with FITC (in red) and mono-clonal anti-HY antibody (C11 F), secondary antibody conjugated to FITC and complement (guinea-pig's serum at 0,8%) (in blue).

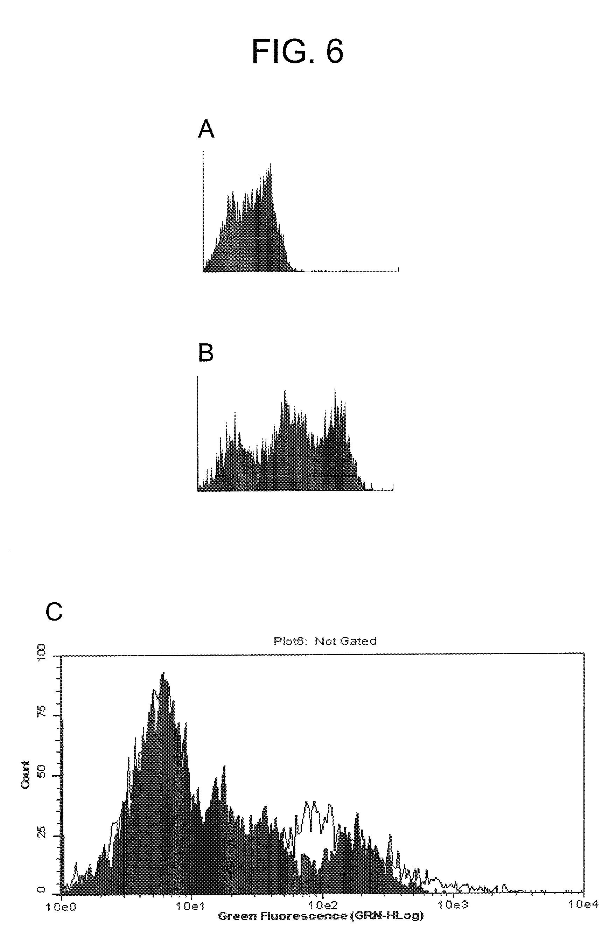

Figure 6B shows the histogram of flow cytometry analysis of spermatozoa marked by the monoclonal anti-HY antibody (C11F) and by the specific goat antibody against mouse antibody and marked with fluorescence. In the figure, three populations of different cells with different fluorescence intensity are shown.

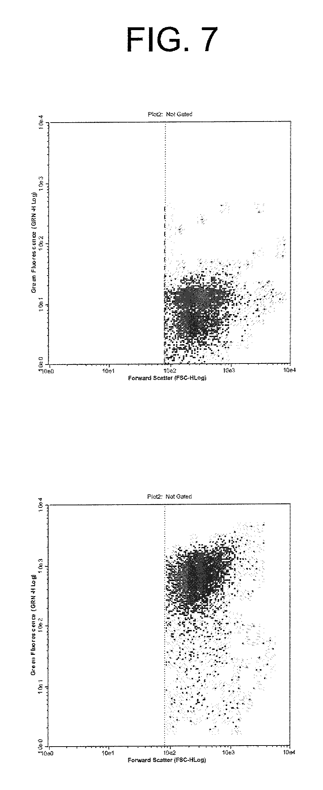

Figure 7A is a dot plot of the flow cytometry analysis of spermatozoa marked only with the monoclonal antibody C11 F, without any fluorescence.
Figure 7B shows a dot plot of the flow cytometry analysis of spermatozoa killed by rapid freezing at (-) 196°C and rapid unfreezing (+) 37°C. This figure shows the hyperfluorescence of the dead cells in the presence of primary and secondary antibodies (monoclonal antibody C11 F and anti-mouse antibody goat antibody marked with FITC, respectively).

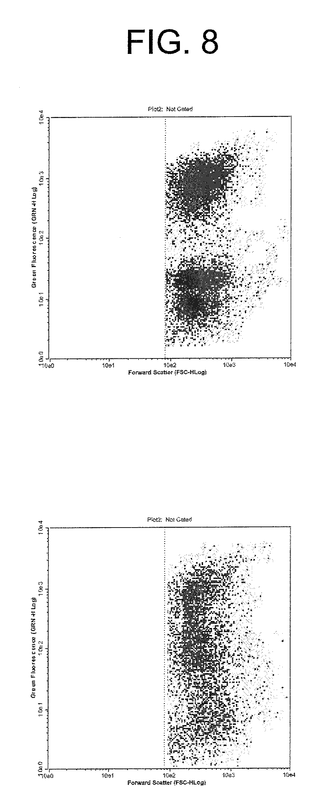

Figures 8A and 8B are dot plots representing the fluorescence of cells incubated in the presence of anti-HY monoclonal antibody and of a secondary antibody marked with fluorescein (FITC). The X axis corresponds to the number of cells, while the Y axis corresponds to the fluorescence intensity.

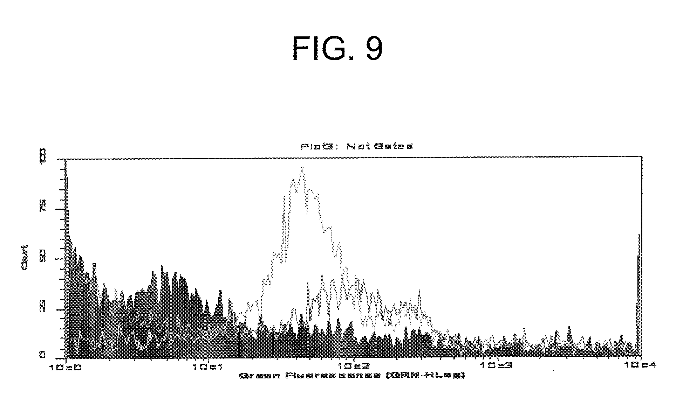

Figure 9 shows the histogram of the flow cytometry analysis of spermatozoa marked with: monoclonal antibody C11 F and secondary antibody conjugated to FITC (in red), monoclonal antibody C11 F, secondary antibody conjugated to FITC and complement (guinea-pig's serum) at 1,0% (in blue) and monoclonal antibody C11 F, secondary antibody conjugated to FITC and complement (guinea-pig's serum) at 0,8% (in green).

### Detailed Description of the Invention

The invention described an immunosexing method for genus-specific enriching of spermatozoa by using a genus-specific monoclonal antibody associated to the action of the classical complement pathway.

The present study has found that the use of limit dilutions of guinea-pig's serum enables one to achieve a dilution in which the alternative complement pathway is no longer functional, but the classical pathway is still completely active. The limit dilution, however, is slightly variable from a serum to another serum in animals of the same species and in animals of different species, for which reason it is necessary to test the serum prior to use.

For the development of this technology, various types of serum of mammals were tested. Also, it was found that the serum exhibited antibodies with non-specific reaction (crossed reaction), which could eliminate the spermatozoa of in a non-specific manner. Sera from bovine, sheep, goats, swine and guinea-pigs were contacted with the spermatozoa, wherein the guinea-pig's serum was the one that exhibited the best results. Guinea-pig's serum exhibited a strong action of the alternative complement pathway, killing 100% of the spermatozoa in about 15 minutes' contact. Agglutination of the spermatozoa by the action of antibodies or another factor that could cause lesion to the membrane was not observed in these cells when in the presence of pre-heated guinea-pig's serum, a fact that was observed with other sera tested, thus being a demonstration that there are no antibodies against membrane protein of bovine spermatozoa in an amount sufficient to cause problems in the immunosexing process in the guinea-pig's serum.

It was further observed that the use of the genus-specific monoclonal antibody on spermatozoa from an ejaculate, associated to a second marked antibody that recognized the monoclonal antibody, when analyzed in flow cytometer, generated the presence of three populations of cells with different marking intensities. The first population, virtually unmarked, composed mainly of spermatozoa of sex opposite to that recognized by the monoclonal antibody; the second population, slightly marked, composed chiefly by spermatozoa of the gender recognized by the monoclonal antibody, and the third population, strongly marked, composed by dead spermatozoa. These data were proven by using the PCR technique on these spermatozoa.

The subsequent analyses demonstrate that the use of a mono-clonal antibody associated to the complement by limit dilution of the serum was capable of reducing by about 50% the live spermatozoa. When using only the monoclonal antibody or the source of the complement in the ideal dilution, the percentage of live spermatozoa was not reduced, this test having been carried out on more than 500 ejaculates. In this same analysis, when the complement was used at a higher concentration than the ideal one, there was a much higher mortality of the spermatozoa, with total elimination thereof, which demonstrated the action of the alternative pathway.

By the analysis in a flow cytometer, it was observed that the use of the ideal dilution of the complement, associated to the monoclonal antibody, is capable of eliminating specifically the population of spermatozoa of medium marking intensity, reducing this population and increasing the population of dead spermatozoa, strongly marked. In this same analysis, when using a higher concentration of complement, one observes a significant reduction of the two populations of live spermatozoa and an increase in the population of dead spermatozoa (figure 9). Ultrasound analysis of cows with about 60 days of pregnancy, inseminated with semen by using the ideal dilution of the complement gives good results, 80% of females in eight bulls in which the amount of complement was ideal. In two other bulls, in which the amount of complement was higher than the ideal one there was no sexing, but a great mortality of spermatozoa took place with 52% of females.

The sexing kit of the invention is based on the production of novel monoclonal antibodies of defined specificity against the H-Y antigen associated to activation of the complement by the classical pathway, the activated proteins of which will bind only to the antigen X antibody complex formed. Another factor determining this kit is the deactivation of protein B responsible for activation of the alternative complement pathway, by using for this purpose the limit dilution of the guinea-pig's serum at a concentration of 0.8%. The evaluation of the classical complement pathway results in the formation of pores in the membrane of the spermatozoa, which, by difference of osmotic pressure, causes liquid from the environment to penetrate, thus killing the spermatozoon.

Although the present invention has been described with regard to specific methods and embodiments, various modifications and alterations can be made without departing from the description.

The following examples illustrate the invention as a whole and should not be considered to limit its scope at all. Such examples are the best mode for carrying out the present invention, particularly Example 3.

### Example 1

### Production of monoclonal antibodies against the H-Y antigen (C11 F antibodies)1.1. Purification of H-Y protein

Bovine spermatozoa of European origin and of Asian origin wee used for purifying the cytoplasm membrane. The proteins were extracted with a buffer of Tris/HCl 20mM with 5% Nonidet P40.

Spermatozoa contained in the Tris/HCl solution were treated by ultrasound (5 times for 30 seconds in ice) using a 3mm probe. The probe was introduced into the atmosphere for one hour at 4°C. Then, the sample was centrifuged for 30 minutes at 14,000 g, and at 3°C. The supernatant was recovered after centrifugation.

The separation of the proteins was made by applying the supernatant collected in a column being 2cm in diameter and 150 cm high. This column was filled with Sephacril S-200 resin (Pharmacy LKB).

The different separated proteins were then recovered by using a Pharmacy LKB 100 fraction collector. The sample was then recovered in 90 different fractions, which were analyzed by spectrophotometry (SPECTROL-ZEISS UV-Vis) at a wavelength of 280nm. The reading pattern used was the Tris/HCl buffer having the composition: 20mM Tris/HCl+0.5% Nonidet P-40.

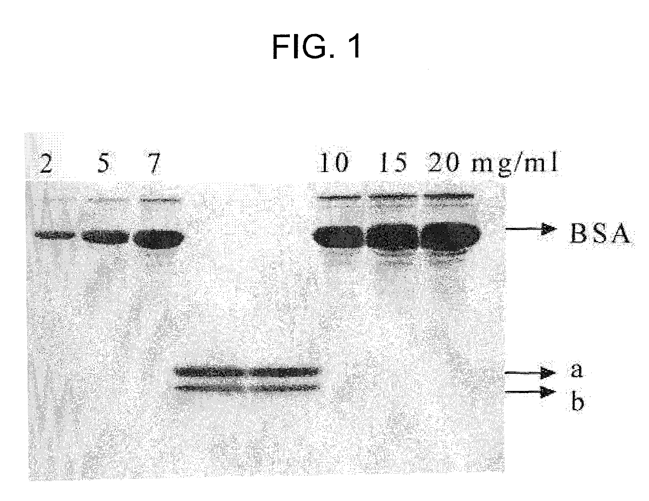

The fractions were subjected to a SDS/PAGE electrophorese field, and the migration was made in a 15% polyacrylamide gel. The proteins with molecular weight close to 19 KDa (HY antigen) were recovered and concentrated. The reading of the amount of proteins recovered was made in comparison with a range of different concentrations of known bovine albumin (see Figure 1).

### 1.2 Immunization

BALB/c mice were immunized with 10µm purified HY antigen, associated to 3µg aluminum hydroxide [AI(OH3)], by intraperitonial (i.p.) route. Two weeks after the first immunization, the animals received two more equal doses of purified HY antigen without adjuvant at weekly intervals, totaling 30µg HY antigen per mouse. Two days after the last injection the animals were killed, and the splenic cells used for fusion.

### 1.3 Fusion

Cells from the spleen of immunized mice were fused with myeloma NS0 cells, by using polyethyleneglycol (PEG) 4000 (Gibco BRL,USA), according to methodology described by Köhler and Milstein (1975). After fusion, the cells were re-suspended in a DMEM-F12 medium (Gibco BRJ, USA) containing 10% bovine fetal serum (SFB), 20µg/ml gentamicin (Gibco BRL, USA), 50µgM β2-mercaptoethano! and selective agent HAT (Hypoxanthine Aminopterin and Thymidine) 50x (Sigma Chemical Co, USA). The cell suspension was seeded on 96-well plates (Corning, USA), which previously contained a monolayer of peritoneal cells from BALB/c mice, so that the concentration of cells in each well would be 2x10⁵ cells. The plates were incubated in an oven at 37°C with 5% CO2 and observed every day under the inverted optical microscope (Axiovert 135 Zeiss, Germany) to follow the growth of cell colonies.

### 1.4 Selection of the Anti-HY Hybridomas

When the colonies of hybridomas reach about 300 cells, the respective supernatants were tested by ELISA test for detecting anti-HY antibodies.

### 1.5 Immunoenzymatic Assay (ELISA) of the Supernatants of the Hybridomas

The ELISA test was carried out by using 10µg/ml of the HY protein diluted in carbonate/bicarbonate buffer 0.05M¹⁴, pH 9.6 (50 µl/well) and incubated overnight at 4°C. After adsorption of the antigen, the plates were washed with PBST and blocked with gelatin at 1% (150µl/well), diluted in PBST an hour, at room temperature. After blocking and new washing, one added 50µl supernatant of culture of the hybridomas per well. After three washes with PBST, one added the mouse anti-IgG conjugate (Southern biotechnologies, USA), marked with peroxidase diluted at 1:2000 (50µl/well), and new incubation was carried out at 37°C for 45 min. This reaction was developed by adding the substrate containing H₂O₂ and orthophenyldiamine - OPD - (Sigma Chemical Co, USA), diluted in acidic buffer. The reaction was interrupted by adding H₂SO₄.3N (50µl/well) and read in wavelength of 490nm in plate spectrophotometer (Dynatech MR 5000).

### 1.6 Hybridoma Cloning

The hybridomas that proved to be positive in the ELISA test were cloned by limiting solution. The cells contained in each positive well were contacted in Neubauer chamber, diluted at 20 cells/ml and seeded in the volume of 100µl per well on 96-well plates containing 100µl of complete DMEM-F-12 medium, supplemented with HT (Hypoxanthine and Thymidine) (Sigma Chemical Co., USA) and pre-conditioned with peritoneal mouse phagocytes. Each clone was subcloned at least twice, before being enlarged for collection of supernatant, production of ascitic liquid or frozen in liquid nitrogen.

### 1.7 Production of Ascitic Liquid

After 7 to 10 days from the injection of 0.5ml Pristane (Sigma Chemical Co. USA) by intraperitoneal route, BALC/c mice were injected by the same route with 2x10⁶ cells of the hybridoma under study, previously washed twice with PBS. The mice were observed every day, and about 10 days after the injection of the cells, the ascitic liquids were collected. The latter were centrifuged at 1000g and the respective supernatants obtained were tittered by ELISA and frozen at -20°C or purified.

The thus obtained ascitic liquids were used for the immunosexaign laboratory tests and also for the field tests.

### Example 2

### Reactivity of monoclonal antibody C11 F with tissues from different organs

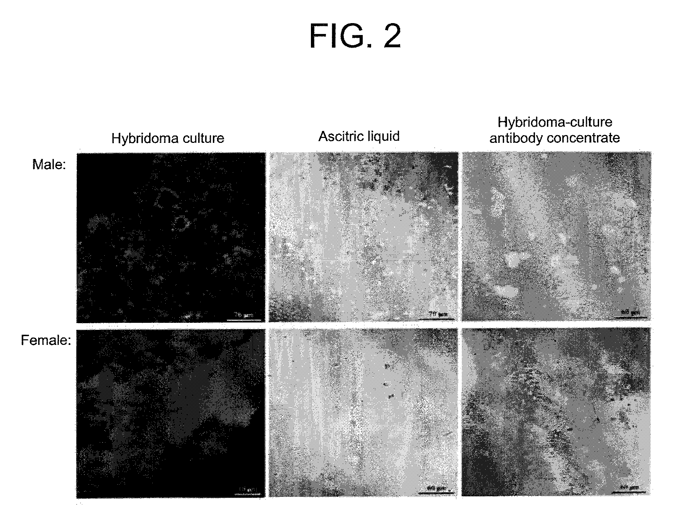

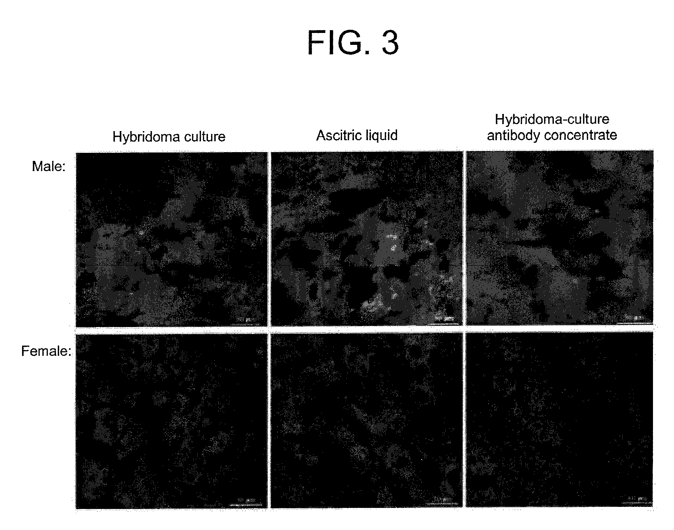

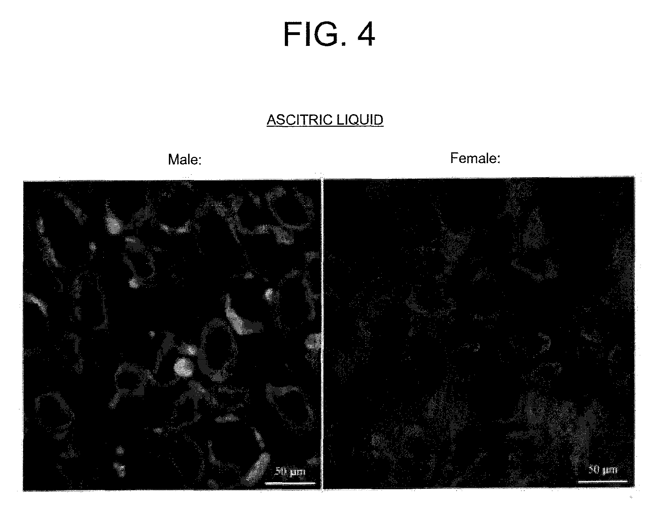

In order to study the specificity of the C11 F antibodies obtained, these were tested on different tissues from male and female bovines, these tissues being the spleen, the liver and the kidney. The reactivity of the mono-clonal antibody with the HY antigen (male-specific) present on the cells of male animals was analyzed by the indirect immunofluorescence technique. The images were obtained by confocal microscopy (LSM 410 Zeiss) (see Figures 2, 3 and 4).

### Example 3

### Elimination of the alternative complement pathway

There are various methods of inhibiting the alternative complement pathway, by using different temperatures (52°C for 30 min; 56°C for 3 min; 50°C for 45 min) or by using antibodies that block the action of specific components of the alternative pathway like protein B.

In the present study it was observed that dilution of the guinea-pig's serum in very low doses can eliminate the alternative complement pathway without affecting the classical pathway. The alternative complement pathway is capable of lysating spermatozoon membrane without the presence of antibodies.

Guinea-pig's serum (complement source) was diluted in Veronal buffer in various concentrations (2%, 1%, 0.8% and 0.4%).

Later, spermatozoa separated from the seminal plasma were incubated with guinea-pig's serum diluted in Veronal buffer in the 4 different dilutions in the absence or presence of HY anti-antigen antibodies - in part of the tubes, 5 mL of ascitic liquid, source fo the HY anti-antigen antibodies, were added - to evaluate the action of different complement activation pathways.

The death of spermatozoa was then determined by optical microscopy. The results were described in Table 1 below.

**Table 1 - Results of the induction of death of spermatozoa by actuation of the complement system either by the alternative pathwa (without adding anti-HY antibody) or by the classical pathway (with addition of anti-HY anti-body) at different concentrations of guinea-pig's serum.**

| Concentration of guinea-pig's serum | Without antibody (actuation of the alternative pathway) | With antibody (actuation of the classical pathway) |
|---|---|---|
| 2% | 100% mortality | 100% mortality |
| 1% | 100% mortality | 100% mortality |
| 0.8% | 0% mortality | 50% mortality |
| 0.4% | 0% mortality | 10% mortality |

The results obtained show that the ideal dilution of this lot of guinea-pig's serum to eliminate the alternative pathway, without affecting the classical complement pathway is of 0.8%. Lower dilutions do not eliminate the alternative pathway and higher dilutions also reduce the classical pathway.

Different lots of serum can have minor variations in the ideal percentage of dilution, for which reason it is necessary to make this test for each lot of serum to be used.

### Example 4

### Analysis by eletrophorese of spermatozoa sexed by the action of monoclonal antibodies and classical complement pathway

Spermatozoa were collected from bulls by using artificial vaginas. The sperm concentration, the total and progressive motility were measured. The spermatozoa were washed with Veronal buffer for removal of seminal plasma by centrifugation at 800 g and with elimination of the supernatant. Then, the spermatozoa were re-suspended with 5ml of monoclonal antibody C11 F, Veronal buffer until 50ml of suspension and 400µl guinea-pig's serum was reached. The suspension was then incubated for one hour at 37°C in a water-bath.

The reaction of the monoclonal antibodies and of the complement over the spermatozoa ends with the death of the spermatozoa recognized by the antibody. Live and dead spermatozoa are then separated by centrifuging the letter in a Percoll gradient (45% and 90%) and centrifuged at 800 g for 30 minutes.

The dead spermatozoa remain in the upper portion of the gradient, while the live spermatozoa migrate to the tube bottom. The two populations of spermatozoa (dead and live) were then collected in a discerning manner, so that no mixing would take place, their cytoplasm membranes were purified as described before and the latter were subjected to a monodimentionsl electrophorese field, and the migration was made in polyacrylamide gel at 16%. The bands were developed by using coomassie blue coloration.

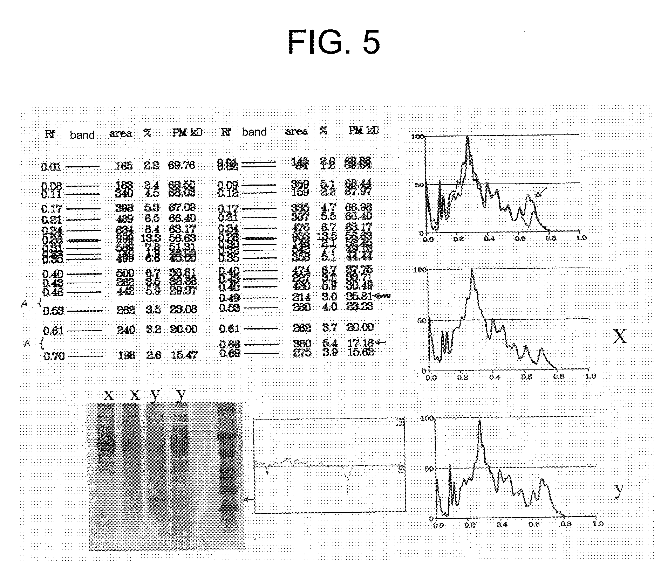

The result of this analysis showed (see figure 5) the presence of two proteins of 17.18 KDa and 25.81 KDa presents in the extracts of the membranes of dead (male) spermatozoa and absent in the extracts of proteins of the (female) spermatozoa. In the extract of the live proteins, one observed the presence of a protein with molecular mass of 52KDa at a concentration of 7,8%, while in the extract of live spermatozoa this protein was at a concentration of 2.1%, therefore four times lower. This protein probably corresponds to the female-specific antigen (HX antigen).

### Example 5

### Analysis by flow cytometry of the marking of the spermatozoa by antibody C11 F (anti-HY).

Flow Cytometry is a technique used for counting, examining and classifying microscopic particles suspended in a flowing liquid medium. It enables the analysis of various parameters simultaneously, being known also as multiparameter flow cytometry. By means of an optical-electronic detection apparatus, analyses of physical and/or chemical characteristics of a mere cell are possible.

### Preparation of the spermatozoa

An ejaculate is washed by centrifugation at 37°C in a TALP buffer at 800 g to remove the seminal plasma by discarding the supernatant.

Later, one makes the marking of the spermatozoa by the monoclonal antibody, by observing the following conditions: 1.10⁶ spermatozoa were incubated with 1µl of ascitic liquid containing monoclonal antibody C11 F in 100µl of TALP buffer at 37°C for 60 minutes.

After this incubation, the spermatozoa are again washed, and 1 ml of TALP buffer is centrifuged for 10 minutes at 800g and at 37°C, the supernatant being discarded.

A buffer containing a second specific antibody for portion Fc of mouse antibody was added and marked with fluorescence.

Incubation was again carried out at 37°C for 60 minutes in the dart in 200µl of TALP buffer in a 1/50 dilution.

After this incubation, the spermatozoa are again washed for removal of the excess of the second antibody, centrifuged at 800g for 10 minutes and at 37°C, and then analyzed by flow cytometry.

The analysis by cytometry was carried out following the parameters given below:
- FSC 25
- Gain of FSC x16
- Flow 0.24 µL/s
- Voltage of the green photomultiplier: 384 V.

The results of the analysis of flow cytometry are shown in Figures 6, 7, 8 and 9.

In Figure 6B, the marking of the spermatozoa with the primary anti-HY antibody and the secondary goat-specific antibody against mouse antibody and marked with fluorescein (FITC) shows clearly 3 populations with different intensity of fluorescence: the first peak, weaker in fluorescence intensity, corresponds to the population of unmarked live cells (females); the second peak corresponds to the population of live cells, which are moderately marked with respect to the preceding cells (males); and the third peak corresponds to the population of strongly marked cells, which are dead cells.

Figure 9 shows, in the red graph, the action of monoclonal antibody C11 F plus the anti-mouse goat antibody marked with FITC. In the green graph guinea-pig's serum at 0.8% was added, and one observed that there was no reduction of the cells of the population of protozoa with lower marking intensity (1 peak), a marked reduction in the population of moderately marked cells (second peak) and a significant increase in strongly marked cells (third peak), showing the action of the complement by the classical pathway. In the blue graph, one used the guinea-pig's serum at 1% concentration, and one can observe the significant reduction of the first two peaks, indiscriminately with a significant increase in the third peak, which shows the action of the complement alternative pathway, keeping both spermatozoa bearing X-chromosomes and spermatozoa bearing Y-chromosomes.

## Claims

1. A method of enriching spermatozoa of mammals bearing X-chromosome, **characterized by** comprising the steps of:
- binding monoclonal antibodies H-Y specific to the H-Y antigen located on the surface of the cytoplasm membrane of the spermatozoa bearing Y-chromosome, wherein said binding induces the activation of the classical complement pathway; and
- eliminating the action of the alternative complement pathway by limiting dilution of the complement source, wherein the complement source is a guinea-pig's serum.

2. A method of enriching spermatozoa of mammals bearing Y-chromosome, **characterized by** comprising the steps of:
- binding monoclonal antibodies H-X specific to the H-X antigen located on the surface of the cytoplasm membrane of the spermatozoa bearing X-chromosome, wherein said binding induces the activation of the classical complement pathway; and
- eliminating the action of the alternative complement pathway by limiting dilution of the complement source, wherein the complement source is a guinea-pig's serum.

3. A method according to claim 1 or 2, **characterized in that** the limiting dilution of the complement source takes place for dilution at a concentration of 0.8%.

## Patentansprüche

1. Verfahren zur Anreichung von X-Chromosom tragenden Spermatozoen von Säugetieren, **gekennzeichnet durch** folgende Schritte:
(a) Binden aus H-Y-Antigen-spezifischen H-Y- monoklonalen Antikörpern, die sich auf der Oberfläche der Y-Chromosom tragenden Zytoplasmamembran befinden, wobei das Binden die Aktivierung des klassischen Pathway-Komplements herbeiführt; und
(b) Beseitigen der Wirkung des alternativen Pathway-Komplements **durch** Einschränkung der Verdünnung der Komplementquelle, wobei die Komplementquelle das Serum eines Versuchstiers ist.

2. Verfahren zur Anreichung von Y-Chromosom tragenden Spermatozoen von Säugetieren, **gekennzeichnet durch** folgende Schritte:
(a) Binden aus H-Y-Antigen-spezifischen H-Y- monoklonalen Antikörpern, die sich auf der Oberfläche der Y-Chromosom tragenden Zytoplasmamembran befinden, wobei das Binden die Aktivierung des klassischen Pathway-Komplements herbeiführt; und
(b) Beseitigen der Wirkung des alternativen Pathway-Komplements **durch** Einschränkung der Verdünnung der Komplementquelle, wobei die Komplementquelle das Serum eines Versuchstiers ist.

3. Verfahren nach dem Anspruch 1 oder 2, **dadurch gekennzeichnet, dass** die Verdünnung der Komplementquelle für Verdünnung bei Konzentration von 0,8% erfolgt.

## Revendications

1. Méthode pour l'enrichissement de spermatozoïdes de mammifères qui portent chromosome-X, **caractérisée en ce qu'**elle comprend les étapes suivantes:
a) liaison des H-Y anticorps monoclonaux spécifiques à l'antigène H-Y situé sur la surface de la membrane de cytoplasme des spermatozoïdes portant chromosome-Y, où ladite liaison provoque l'activation du chemin de complément classique; et
b) élimination de l'action du chemin de complément alternatif par la limitation de la dilution de la source complément, où la source complément est le sérum d'un cochon d'Inde.

2. Méthode pour l'enrichissement de spermatozoïdes de mammifères qui portent chromosome-Y, **caractérisée en ce qu'**elle comprend les étapes suivantes:
a) liaison des H-Y anticorps monoclonaux spécifiques à l'antigène H-Y situé sur la surface de la membrane de cytoplasme des spermatozoïdes portant chromosome-X, où ladite liaison provoque l'activation du chemin de complément classique; et
b) élimination de l'action du chemin de complément alternatif par la limitation de la dilution de la source complément, où la source complément est le sérum d'un cochon d'inde.

3. Méthode selon la révendication 1 ou 2, **caractérisée en ce que** la limitation de la dilution de la source complément a lieu pour une dilution à la concentration de 0,8%.
